# EUROPÄISCHE PATENTSCHRIFT

(11) **EP 1 330 203 B1**
(45) Veröffentlichungstag und Bekanntmachung des Hinweises auf die Patenterteilung: **12.09.2007**
(21) Anmeldenummer: 01992534.6
(22) Anmeldetag: 31.10.2001
(51) Int. Cl.: A61B 19/00

(54) **VORRICHTUNG ZUM HALTEN UND POSITIONIEREN EINES ENDOSKOPISCHEN INSTRUMENTS**
DEVICE FOR HOLDING AND POSITIONING AN ENDOSCOPIC INSTRUMENT
DISPOSITIF DE MAINTIEN ET DE POSITIONNEMENT D'UN INSTRUMENT ENDOSCOPIQUE

(30) Priorität: 03.11.2000 DE 10055293
(43) Veröffentlichungstag der Anmeldung: 30.07.2003
(73) Patentinhaber: Karl Storz GmbH & Co. KG, 78532 Tuttlingen (DE)
(72) Erfinder: IRION, Klaus, M., 78576 Liptingen (DE); BÖHM, Ralph, 78351 Bodman-Ludwigshafen (DE)
(74) Vertreter: Heuckeroth, Volker
(86) Internationale Anmeldenummer: PCT/EP2001/012650
(87) Internationale Veröffentlichungsnummer: WO 2002/036030

(56) Entgegenhaltungen:
- US-A- 2 697 433
- US-A- 3 308 675
- US-A- 5 201 742
- US-A- 5 767 839

## Beschreibung

Die Erfindung betrifft eine Vorrichtung zum Halten und Positionieren eines durch eine Körperoberfläche eines Patienten eingeführten endoskopischen Instruments gemäß dem Oberbegriff des Anspruchs 1.

Eine derartige Vorrichtung ist aus dem Dokument US-A-2 697 433 bekannt.

Eine derartige Vorrichtung wird im Rahmen der minimal-invasiven Chirurgie verwendet. Bei der minimal-invasiven Chirurgie wird ein Instrument, worunter im Rahmen der vorliegenden Erfindung ein Arbeitsinstrument wie bspw. eine Schere, Zange zum Präparieren von Gewebe als auch ein Endoskop verstanden wird, durch eine Inzision in der Körperoberfläche in das Operationsgebiet eingeführt, um einen chirurgischen Eingriff unter endoskopischer Sichtkontrolle durchzuführen. Das Instrument wird dabei üblicherweise durch einen Trokar, der in die Inzision eingesetzt ist, in das Körperinnere eingeführt.

Insbesondere, wenn es sich bei dem Instrument um ein Endoskop handelt, ist es mitunter erforderlich, das Endoskop in verschiedenen Kippstellungen relativ zur Körperoberfläche zu positionieren, um alle Bereiche des Operationsgebiets einsehen zu können.

Das Positionieren des Instruments bzw. des Trokars wurde herkömmlich vom Assistenzpersonal durchgeführt. Wünschenswert ist es jedoch, wenn derartige einfache Tätigkeiten, wie das Positionieren des Instruments, von einer steuerbaren Halte- und Positioniervorrichtung übernommen werden, wodurch Assistenzpersonal eingespart und darüber hinaus ein exakteres Positionieren des Instruments ermöglicht werden könnte.

So wäre die Halterung und die Nachführung des Endoskops, das zur Videobilderfassung die Kamera trägt, über ein Endoskophalte- bzw. Führungssystem sinnvoll. Das Halten und Positionieren eines Endoskops bzw. Instruments ist bspw. mit einer aus der US 4,573,452 bekannten Vorrichtung möglich. Diese Vorrichtung weist ein wahlweise spannbares kabelartiges Element auf, das zum Bewegen des Endoskops entspannt wird, wonach das Endoskop manuell in die gewünschte Position gebracht werden kann, und anschließend zu einer starren Struktur gespannt wird, um das Endoskop in der eingestellten Position zu fixieren. Nachteilig an dieser bekannten Vorrichtung ist, daß die Positionierung, d.h. die Lageveränderung, des Endoskop ausschließlich manuell erfolgen kann.

Die aus dem eingangs genannten Dokument US-A-2 697 433 bekannte Halte- und Positioniervorrichtung, von der die vorliegende Erfindung ausgeht, weist zwei um 90° zueinander versetzt angeordnete bogenförmige Elemente auf, die um zwei senkrecht zueinander stehende Schwenkachsen verschwenkbar sind. Mit dieser Halte- und Positioniervorrichtung können Führungsdrähte, die beim Nageln des Oberschenkelhalses verwendet werden, geführt werden.

Ein weiteres manuell einstellbares Halte- und Positioniersystem ist aus dem Dokument US-A-5 810 712 bekannt, das einen Halter in Form eines Rings mit einer darin kardanisch gelagerten Kugel als Aufnahme für das Instrument aufweist. Die kardanisch aufgehängte Kugel kann in der gewünschten Kippstellung fixiert werden.

Es sind aber bereits auch motorisch angetriebene Vorrichtungen zum Halten und Positionieren eines Instruments bekannt, bspw. aus US-A-5 184 601 oder aus US-A-5 766 126. Diese Vorrichtungen ermöglichen es nicht nur, das Endoskop aufzunehmen und in einer Position zu fixieren, sondern während der Operation auch den dabei verwendeten Arbeitsinstrumenten intrakorporal nachzuführen, entweder über eine direkte Ansteuerung von außen oder automatisch durch Erkennung der Instrumentenspitzen der Bildverarbeitung und entsprechender Steuerung des Führungssystems, oder durch Lage- und Richtungserkennung der Instrumente über die Detektion durch Positionssensoren und entsprechender Steuerung der Halte- und Positioniervorrichtung.

Nachteilig an diesen intelligenten Halte- und Positioniervorrichtungen ist jedoch deren aufwendiger und komplizierter Aufbau und insbesondere ihre Baugröße, die zu einer erheblichen Verdeckung des Operationsfeldes führt. Des weiteren sind diese bekannten Vorrichtungen aufwendig zu installieren und einer Reinigung nur schwer zugänglich. Aufgrund ihres komplizierten Aufbaus und der Verwendung empfindlicher Bauteile sind diese Vorrichtungen nicht in einem Autoklaven sterilisierbar und bedürfen daher aus Hygienegründen während der Durchführung der Operation eines sterilen Folienüberzugs. Des weiteren sind diese Vorrichtungen kostenaufwendig und in der Regel aufgrund ihrer schweren und massiven Ausführung nicht transportabel, so daß sie nicht nacheinander an verschiedenen Einsatzorten verwendet werden können.

Aufgrund dieser Nachteile eignen sich diese bekannten Vorrichtungen nicht bei einfachen und kurzen minimal-invasiven Eingriffen, da die Installations- und Vorbereitungszeiten für diese bekannten Vorrichtungen in keiner Relation zur Dauer der Operation stehen.

Das Dokument DE 94 15 039 U1 beschreibt eine Vorrichtung zur Führung chirurgischer Instrumente, bspw. eines Trokares, bei dem die beiden bügelförmigen Elemente mit ihren jeweils beiden Endabschnitten auf jeweils einer kreissegmentförmigen Führungsbahn über Laufrollen verfahrbar sind. Diese vergleichsweise konstruktiv einfache Vorrichtung ermöglicht dem in der Vorrichtung mit seinem Schaft eingespannten Instrument den Bewegungsraum einer rechteckigen Doppelpyramide, deren gegeneinanderweisende Spitzen in einem invarianten Punkt zusammenfallen, der auf der Schaftachse des Instrumentes liegt, und wobei sich beide Enden des Schaftes auf je einer Kugelsektorfläche bewegen, deren Mittelpunkte im invarianten Punkt liegen. Die Führung der beiden bügelförmigen Elemente über Rollen auf gekrümmten Führungsbahnen hat jedoch verschiedene Nachteile.

Aufgrund der Wölbung der Führungsbahnen besitzt der Rahmen dieser Vorrichtung eine beträchtliche Bauhöhe, die nachteiligerweise bewirkt, daß der invariante Punkt der Instrumentenbewegung weit oberhalb der Körperoberfläche liegt. Des weiteren ist es schwierig, die Laufrollen mit einem motorischen Antrieb zu versehen, wenn dies für eine Automatisierung der Instrumentenpositionierung gewünscht ist. Des weiteren weist diese bekannte Vorrichtung aufgrund der Führungsrollen und Führungsbahnen sehr viele Nischen auf, in denen sich Verunreinigungen ansammeln können, die nur schwer zu beseitigen sind. Des weiteren ist es bei dieser bekannten Vorrichtung erforderlich, den Rahmen aufgrund der Führungsbahnen rechteckig auszuführen, was einen erhöhten Platzbedarf erfordert.

Eine weitere Halte- und Positioniervorrichtung gemäß dem Oberbegriff des Anspruchs 1 ist aus dem Dokument US-A-5 767 839 bekannt. Diese Halte- und Positioniervorrichtung wird für die Simulation chirurgischer Eingriffe verwendet, wobei dort anstelle eines realen Instruments ein Joystick verwendet wird.

Weiterhin ist aus US-A-5 330 485 eine Instrumentenführungsvorrichtung zur Führung von Instrumenten für Hirnoperationen bekannt. Diese bekannte Vorrichtung weist zwei unterschiedlich gekrümmte bügelförmige Elemente auf, die an einer gemeinsamen Schwenkachse befestigt und um diese verschwenkbar sind. Bei dieser Vorrichtung kann das zu führende Instrument nicht um einen invarianten Punkt bewegt werden.

Ferner ist aus dem Dokument US-A-5 201 742 eine Halte- und Positioniervorrichtung für ein chirurgisches Instrument bekannt. Diese Vorrichtung weist einen Basisring zum Plazieren der Vorrichtung auf der Körperoberfläche auf. Diese Vorrichtung weist am Führungsschaft ein ringförmiges Element auf, das das Ausströmen eines Insufflationsgases aus dem Körperhohlraum während des endoskopischen Behandlungsvorganges verhindert.

Der Erfindung liegt die Aufgabe zugrunde, eine Vorrichtung der eingangs genannten Art bereitzustellen, mit der die Durchführung eines endoskopischen Eingriffs vereinfacht wird.

Erfindungsgemäß wird diese Aufgabe hinsichtlich der eingangs genannten Vorrichtung dadurch gelöst, daß Mittel zum Anheben der Körperoberfläche in dem von den bügelförmigen Elementen aufgespannten Raum vorgesehen sind.

Bei der erfindungsgemäßen Vorrichtung wird demnach der Halter für das Instrument durch zwei überkreuz angeordnete bügelförmige Elemente gebildet, die aufgrund ihrer Schwenkbarkeit um die erste bzw. zweite Schwenkachse als kardanische Aufhängung für das Instrument wirken. Der konstruktive Aufbau der erfindungsgemäßen Vorrichtung ist besonders einfach, weil die kardanische Aufhängung des Instruments lediglich die beiden verschwenkbaren bügelförmigen Elemente benötigt. Die erfindungsgemäße Vorrichtung ermöglicht wie bei den einfachen bekannten Systemen sowohl eine manuelle Positionierung, eignet sich jedoch auch für eine über motorische Antriebe gesteuerte Positionierung, indem bspw. die bügelförmigen Elemente mittels motorischer Antriebe verschwenkt werden, die ihre Steuersignale von einer entsprechenden Steuereinheit erhalten. Die erfindungsgemäße Vorrichtung ist darüber hinaus leicht zu installieren, weil sie unmittelbar auf der Körperoberfläche um die Inzision herum aufgesetzt werden kann. Da der Halter im wesentlichen lediglich durch die beiden bügelförmigen Elemente gebildet ist, wird das Operationsfeld auch nicht wesentlich verdeckt. Die erfindungsgemäße Vorrichtung läßt sich darüber hinaus einfach reinigen und kann bei entsprechender Materialwahl für die bügelförmigen Elemente auch autoklaviert werden. Ein Instrumentenwechsel läßt sich leicht durchführen, da das Instrument zum Einsetzen in die Vorrichtung lediglich am Überlapp der beiden bügelförmigen Elemente angebracht werden muß. Durch die Ausgestaltung des Halters in Form zweier um etwa 90° zueinander versetzter bügelförmiger Elemente lassen sich beliebige Kippstellungen relativ zur Körperoberfläche realisieren.

Besonders leicht gestaltet sich das Anbringen des Instruments am Überlappbereich der bügelförmigen Elemente, wenn beide bügelförmigen Elemente jeweils eine sich längs der Elemente erstreckende Öffnung aufweisen, und der Überlappbereich der beiden Öffnungen als Führung bzw. Durchführung für das Instrument dient, weil das Instrument dann einfach in den Überlappbereich der Öffnungen eingeführt werden kann.

Um den sogenannten invarianten Punkt der kardanischen Aufhängung vollständig in die Inzision hineinzuverlegen, sind Mittel zum Anheben der Körperoberfläche in den durch die bügelförmigen Elemente aufgespannten Raum vorgesehen.

Diese Mittel können mechanischer Art sein, bspw. ein mechanisches Hebesystem, das einen Greifer oder Gewebehaken aufweist, der durch die Inzision unter die Körperoberfläche greift und die Körperoberfläche anhebt, oder können in einem von außen auf die Körperoberfläche applizierten Unterdruck zum Anheben der Körperoberfläche und damit zur Verlagerung des invarianten Punktes der Schwenkbewegung in die Inzision hinein bestehen.

In einer bevorzugten Ausgestaltung befinden sich die erste Schwenkachse und die zweite Schwenkachse in unmittelbarer Nähe der Körperoberfläche.

Wenn ein Instrument, bspw. ein Endoskop oder ein Trokar durch eine Inzision in das Körperinnere eingeführt wird, liegt der Drehpunkt des Instruments hinsichtlich der Kippstellungen relativ zur Körperoberfläche etwa in der Inzision. Durch die vorstehend genannte Maßnahme wird nun erreicht, daß auch bei Verwendung der erfindungsgemäßen Vorrichtung dieser "natürliche" Drehpunkt des Instruments innerhalb der Inzision, der bei der kardanischen Aufhängung durch den Schnittpunkt der beiden Schwenkachsen gebildet wird, nahezu erhalten bleibt.

In einer weiteren bevorzugten Ausgestaltung sind die bügelförmigen Elemente als gekrümmte flache Stäbe ausgebildet.

Diese Maßnahme trägt vorteilhafterweise dazu bei, daß die erfindungsgemäße Vorrichtung im wesentlichen kein Sicht- oder Zugangshindernis auf das Operationsfeld darstellt.

In einer weiteren bevorzugten Ausgestaltung sind die bügelförmigen Elemente mit ihren Endabschnitten an einem ringförmigen Basiselement befestigt, das auf die Körperoberfläche auflegbar ist, wobei die Schwenkachsen im Basiselement liegen.

Aufgrund des Basiselements läßt sich die erfindungsgemäße Vorrichtung als kompakte Einheit in einfach zu handhabender Weise auf der Körperoberfläche um die Inzision herum positionieren, wobei das Basiselement vorteilhafterweise für eine im wesentlichen verkippfreie Auflage der erfindungsgemäßen Vorrichtung auf der Körperoberfläche sorgt.

Dabei ist es bevorzugt, wenn das Basiselement schwerer ist als die bügelförmigen Elemente.

Durch diese Maßnahme wird der Schwerpunkt der erfindungsgemäßen Vorrichtung vorteilhafterweise so weit wie möglich nahe an die Körperoberfläche verlagert, wodurch die erfindungsgemäße Vorrichtung besonders stabil gegen auf die Vorrichtung wirkende Kippkräfte bzw. Kippmomente ist, die durch das Gewicht und die Winkellage des Instruments verursacht werden.

Um die erfindungsgemäße Vorrichtung noch besser gegen die vorgenannten Kippkräfte bzw. Kippmomente zu sichern, ist es in einer weiteren bevorzugten Ausgestaltung vorgesehen, daß das Basiselement auf der Körperoberfläche befestigbar ist.

In bevorzugten Ausgestaltungen kann die Befestigung des Basiselements auf der Körperoberfläche klebend erfolgen, bspw. durch ein doppelseitiges Klebeband, oder wie in einer noch weiteren bevorzugten Ausgestaltung angegeben ist, durch Anlegen eines Vakuums, über das das Basiselement an die Körperoberfläche angesaugt wird.

In einer weiteren bevorzugten Ausgestaltung ist das Basiselement mit einem ortsfesten Tragrahmen verbunden.

Diese Maßnahme kann vorteilhafterweise störende Bewegungen der Bauchdecke, die bspw. durch Atmung bedingt sind, kompensieren und ebenso Kräfte aufnehmen, die über das Gewicht und die Winkellage des Instruments verursacht werden, um die Vorrichtung ortsfest auf der Körperoberfläche zu fixieren.

In einer weiteren bevorzugten Ausgestaltung sind die bügelförmigen Elemente etwa halbkreisförmig ausgebildet und erstrecken sich die Öffnungen über nahezu die gesamte Länge der bügelförmigen Elemente.

Diese Maßnahme hat den Vorteil, daß sich das Instrument mit der erfindungsgemäßen Vorrichtung in nahezu dem gesamten Raumwinkelbereich von +90° bis -90° um beide Schwenkachsen bezüglich der Vertikalen positionieren läßt. Mit anderen Worten, werden durch diese Ausgestaltung alle Schwenkwinkel im Halbraum oberhalb der Körperoberfläche abgedeckt.

In der Praxis ist es bevorzugt, wenn die Verkippung der bügelförmigen Elemente auf weniger als ± 90°, vorzugsweise auf ±70°, bezüglich der Vertikalen begrenzbar ist.

Eine derartige Begrenzung der Schwenkbereiche der beiden bügelförmigen Elemente kann auf mechanische Weise erfolgen, oder im Falle von einer motorischen Steuerung der Verschwenkung der bügelförmigen Elemente über eine entsprechende Endabschaltung der motorischen Antriebe bei Erreichen des Grenzwinkels.

In einer weiteren bevorzugten Ausgestaltung ist für beide bügelförmigen Elemente ein motorischer Antrieb vorgesehen, um die bügelförmigen Elemente um die erste bzw. zweite Schwenkachse zu verschwenken.

Wie bereits erwähnt, eignet sich die erfindungsgemäße Vorrichtung nicht nur für eine manuelle Positionierung des Instruments, sondern es lassen sich an den bügelförmigen Elementen in geeigneter Weise motorische Antriebe anbringen, um eine gesteuerte Positionierung des Instruments durchzuführen. Eine gesteuerte motorische Betätigung der bügelförmigen Elemente hat den Vorteil, daß kein Assistenzpersonal benötigt wird und die Positionierung noch feiner abgestimmt werden kann, und daß die Verschwenkung des Instruments anhand von Signalen, bspw. von Positionssensoren, die die Lage der Instrumentenspitze verfolgen, erfolgen kann.

In diesem Sinne ist es bevorzugt, wenn die motorischen Antriebe jeweils Positionsdetektoren aufweisen, so daß eine voll automatische Steuerung der kardanischen Aufhängung des Instruments erreicht wird.

In einer weiteren bevorzugten Ausgestaltung sind die motorischen Antriebe innerhalb des von dem bügelförmigen Elementen aufgespannten Raums angeordnet.

Hierbei wird der Vorteil einer noch kompakteren Bauweise erreicht, und es können elektrische Komponenten außerhalb der durch die bügelförmigen Elemente aufgespannten räumlichen Struktur vermieden werden.

In einer weiteren bevorzugten Ausgestaltung ist der Halter so ausgebildet, daß das Instrument in der Führung durch die bügelförmigen Elemente um seine Längsachse verdrehbar und/oder in Richtung seiner Längsachse verschiebbar ist.

Diese Maßnahme hat den Vorteil, daß neben den durch die kardanische Aufhängung mittels der beiden bügelförmigen Elemente vermittelten zwei Freiheitsgrade um die beiden Schwenkachsen das Instrument zwei weitere, vorzugsweise steuerbare Positionierungsfreiheitsgrade besitzt, wodurch eine noch genauere Positionierung des Instruments ermöglicht wird. Im Fall, daß das Instrument ein Endoskop ist, kann durch Vor- und Zurückbewegen des Endoskops eine Vergrößerung bzw. Verkleinerung des Bildfeldes in der Art eines Zooms bewirkt werden, oder auch eine Änderung des Blickpunktes vorgenommen werden. Durch Drehung des Endoskops um seine Längsachse kann das auf dem Videobildschirm zur Darstellung gebrachte Bild des Operationsgebietes aufgerichtet werden. Im Fall, daß das Endoskop einen Blickwinkel schräg zu seiner Längsachse besitzt, kann durch Drehung des Endoskops um seine Längsachse der Bildausschnitt variiert werden.

Hierbei ist es bevorzugt, wenn für die Verdrehbarkeit des Instruments um die Längsachse und/oder für die Verschiebbarkeit des Instruments in Richtung der Längsachse zumindest ein motorischer Antrieb am Halter angeordnet ist.

Mit dieser Maßnahme können auch die beiden zuvor genannten Freiheitsgrade der Bewegung des Instruments anstatt manuell rechnergestützt gesteuert werden.

Weiterhin ist es bevorzugt, wenn der zumindest eine motorische Antrieb für die Verdrehung des Instruments bzw. für die Verschiebung des Instruments indirekt auf das Instrument wirkt.

Hierbei ist von Vorteil, daß Sterilitäts- und Dichtigkeitsprobleme, die möglicherweise auftreten, wenn die motorischen Antriebe direkt auf das Instrument einwirken, vermieden werden. In diesem Sinne kann der zumindest eine motorische Antrieb über eine Magnetkupplung indirekt auf das Instrument wirken.

In einer weiteren bevorzugten Ausgestaltung sind die motorischen Antriebe als Schrittmotoren ausgeführt.

Hierbei ist von Vorteil, daß die gewünschte Position in reproduzierbarer Weise angefahren werden kann. In Verbindung mit dem Vorsehen von Positionsdetektoren, bspw. in Form von Winkelencodern, kann die aktuelle Position des Instruments erfaßt, gespeichert und wieder reproduzierbar angefahren werden.

In einer weiteren bevorzugten Ausgestaltung ist für die motorischen Antriebe eine Sicherheitsabschaltung vorgesehen, derart, daß durch eine im Instrument induzierte Meßspannung bei Berührung des Instruments mit Gewebe im Körperinneren ein Meßstrom fließt, der eine Abschaltung der motorischen Antriebe bewirkt.

Durch diese Maßnahme wird den an die Sicherheit der erfindungsgemäßen Vorrichtung zu stellenden Anforderungen in hohem Maße genüge getan, um unerwünschte Kollisionen des Instruments mit unbeteiligtem Gewebe und dadurch verursachte Verletzungen zu vermeiden.

In einer weiteren bevorzugten Ausgestaltung weist die erfindungsgemäße Vorrichtung eine Ansteuereinheit zur fortlaufenden Erfassung der Positionen der Instrumente und zur Steuerung der motorischen Antriebe auf.

Durch diese Maßnahme läßt sich das in der Vorrichtung aufgenommene Instrument elektronisch gesteuert positionieren. Die Positionierungseingabe kann bspw. interaktiv durch Joysticks, Spracheingabe oder über die Richtungsinformation eines vollautomatischen Trackings der Arbeitsinstrumente über eine spezielle Echtzeitbildverarbeitung erfolgen.

Eine weitere Vorrichtung, die sich als Halte- und Positioniervorrichtung für ein Instrument eignet, ist in den am selben Tag für dieselbe Anmelderin eingereichten zwei deutschen Patentanmeldungen mit dem Titel "Simulationsvorrichtung mit zumindest zwei Bewegungsfreiheitsgraden für ein Instrument" offenbart, deren Offenbarung hierin ausdrücklich aufgenommen wird.

Als Modifikation der dort beschriebenen Simulationsvorrichtung ist es bevorzugt, wenn das Teil der kardanischen Aufhängung der dortigen Vorrichtung bildende kugelförmige Element weggelassen wird. Das dort beschriebene Doppelkegelradgetriebe für die Freiheitsgrade der Bewegung des Instruments um die und in Längsrichtung des Instruments kann auch bei der vorliegenden Vorrichtung vorgesehen sein.

Weitere Merkmale und Vorteile ergeben sich aus der nachfolgenden Beschreibung und der beigefügten Zeichnung.

Es versteht sich, daß die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in der Zeichnung dargestellt und wird in der nachfolgenden Beschreibung näher erläutert. Es zeigen:
- Fig. 1: eine Vorrichtung zum Halten und Positionieren eines Instruments in einer teilweise geschnittenen ersten Seitenansicht;
- Fig. 2: die Vorrichtung in Fig. 1 in einer gegenüber Fig. 1 um 90° gedrehten, teilweise geschnittenen Seitenansicht;
- Fig. 3: die Vorrichtung in Fig. 1 und 2 in einer Draufsicht, wobei gegenüber Fig. 1 Teile weggelassen wurden;
- Fig. 4: eine weitere Draufsicht auf die Vorrichtung in Fig. 1 bis 3 in einer gegenüber Fig. 1 bis 3 veränderten Betriebsstellung; und
- Fig. 5: die Vorrichtung in Fig. 1 bis 4 mit einem weiteren Aspekt der Erfindung.

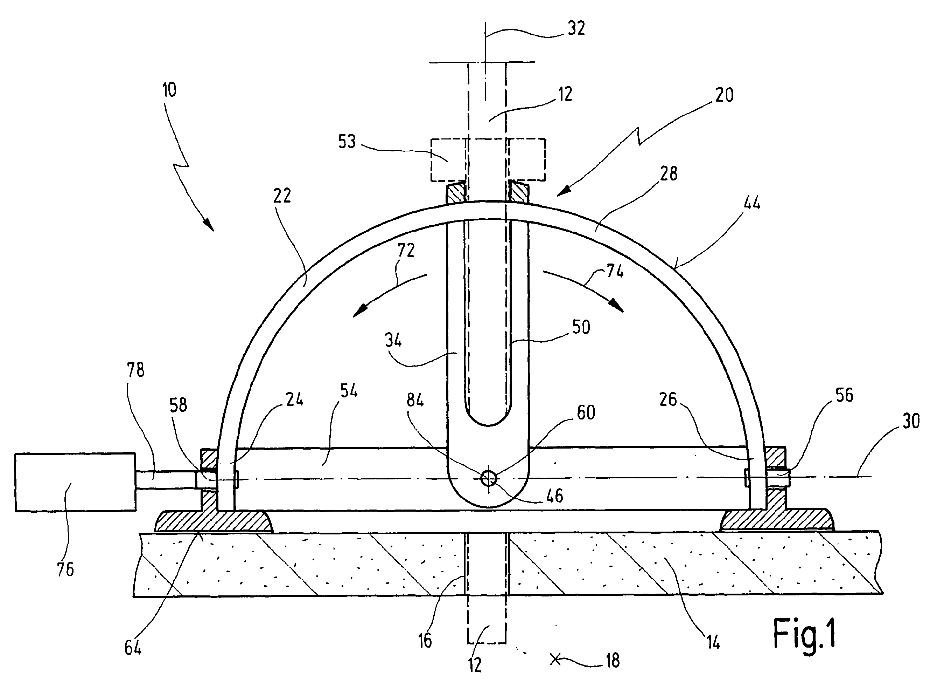

In Fig. 1 bis 5 ist eine mit dem allgemeinen Bezugszeichen 10 versehene Vorrichtung zum Halten und Positionieren eines Instruments 12 (Fig. 1, 2 und 5) dargestellt.

Das Instrument 12 ist durch eine Körperoberfläche 14, bspw. durch die Bauchdecke, durch eine Inzision 16 in der Körperoberfläche 14 hindurch in das Körperinnere 18 eines Patienten eingeführt. Die Vorrichtung 10 wird dabei im Rahmen eines minimal-invasiven Eingriffes zum Halten und Positionieren des Instruments 12 verwendet.

Das Instrument 12 ist ein endoskopisches Instrument, bspw. ein Endoskop selbst oder ein Arbeitsinstrument, wie bspw. eine Schere, Zange oder dgl., wobei das Instrument 12 ggf. durch einen Trokar in das Körperinnere 18 eingeführt ist, und der Trokar dann entsprechend durch die Vorrichtung 10 gehalten und positioniert wird, während das Instrument 12 dann durch den Trokar geführt ist.

Die Vorrichtung 10 weist einen Halter 20 für das Instrument 12 auf, der so ausgebildet ist, daß das Instrument 12 relativ zur Körperoberfläche 14 in verschiedenen räumlichen Kippstellungen positionierbar ist.

Dazu weist der Halter 20 ein erstes bügelförmiges Element 22 auf, das zwei Endabschnitte 24 und 26 aufweist. Die beiden Endabschnitte 24 und 26 sind der Körperoberfläche 14 zugewandt angeordnet.

Ein mittlerer Abschnitt 28 zwischen den Endabschnitten 24 und 26 des ersten bügelförmigen Elements 22 ist von der Körperoberfläche 14 abgewandt, d.h. ist von dieser weiter beabstandet als die Endabschnitte 24 und 26.

Das erste bügelförmige Element 22 ist um eine erste Schwenkachse 30, die durch beide Endabschnitte 24 und 26 hindurchgeht, verschwenkbar.

Der Halter 20 weist weiterhin ein zweites bügelförmiges Element 34 auf, das zwei Endabschnitte 36 und 38 aufweist. Die Endabschnitte 36 und 38 sind der Körperoberfläche 14 zugewandt. Bezüglich der Endabschnitte 24 und 26 des ersten bügelförmigen Elements 22 sind die Endabschnitte 36 und 38 um etwa 90° versetzt angeordnet.

Ein mittlerer Abschnitt 40 des zweiten bügelförmigen Elements 34 zwischen den Endabschnitten 36 und 38 ist von der Körperoberfläche 14 abgewandt, d.h. ist von der Körperoberfläche 14 weiter beabstandet als die Endabschnitte 36 und 38.

Dabei erhebt sich der mittlere Abschnitt 40 des zweiten bügelförmigen Elements 34 im wesentlichen im gleichen Abstand von der Körperoberfläche 14 wie der mittlere Abschnitt 28 des ersten bügelförmigen Elements 22. Genauer gesagt ist eine Innenseite 42 des zweiten bügelförmigen Elements 34 gleich weit von der Körperoberfläche 14 beabstandet wie eine Außenseite 44 des ersten bügelförmigen Elements 22, so daß sich die Innenseite 42 und die Außenseite 44 leicht berühren.

Das zweite bügelförmige Element 34 ist um eine zweite Schwenkachse 46, die durch beide Endabschnitte 36 und 38 hindurchgeht, verschwenkbar. Die zweite Schwenkachse 46 verläuft demnach etwa rechtwinklig zur ersten Schwenkachse 30.

Das erste bügelförmige Element 22 weist eine sich entlang des ersten bügelförmigen Elements 22, genauer gesagt entlang des mittleren Abschnitts 28 erstreckende Öffnung 48 in Form eines Langloches auf.

Das zweite bügelförmige Element 34 weist eine sich entlang des zweiten bügelförmigen Elements 34 erstreckende Öffnung 50 auf, die ebenfalls als Langloch ausgebildet ist.

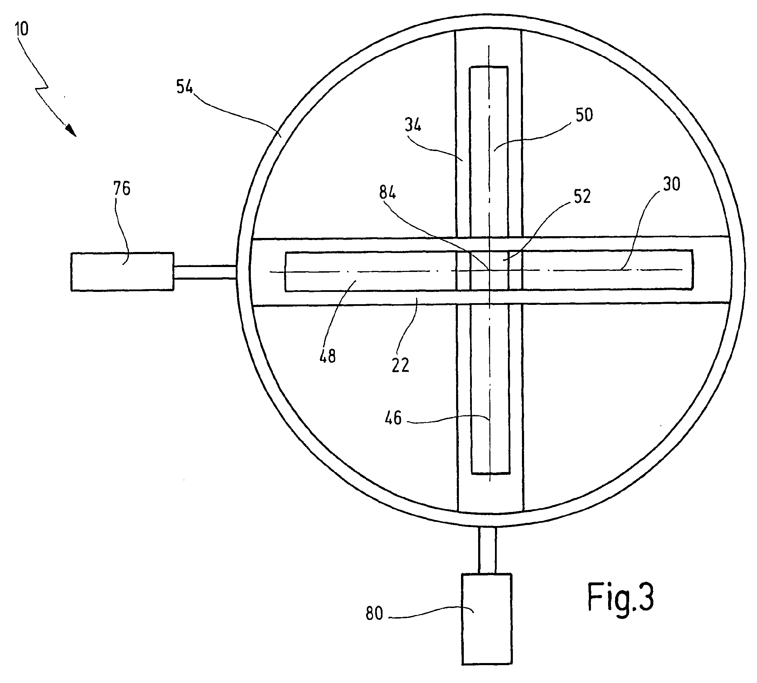

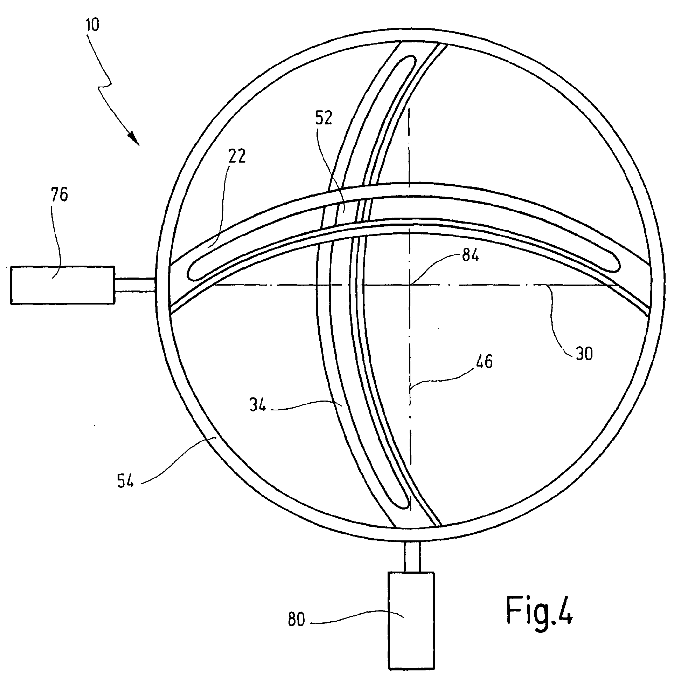

Ein Überlappbereich 52 der beiden Öffnungen 50 und 48 (vgl. Fig. 3) dient als Führung für das Instrument 12, d.h. das Instrument 12 ist in der Führung aufgenommen. In Fig. 3 und 4 ist das Instrument 12 der Übersichtlichkeit halber weggelassen worden.

Das erste bügelförmige Element 22 und das zweite bügelförmige Element 34 sind als gekrümmte flache Stäbe ausgebildet, wobei diese im gezeigten Ausführungsbeispiel etwa halbkreisförmig ausgebildet sind. Der Radius der Außenseite 44 des ersten bügelförmigen Elements 22 entspricht dabei dem Radius der Innenseite 42 des zweiten bügelförmigen Elements 34. Die Öffnungen 48 und 50 erstrecken sich nahezu über die gesamte Länge der bügelförmigen Elemente 22 und 34.

Das Instrument 12 ist in der durch den Überlappbereich 52 gebildeten Durchführung mittels eines Einsatzes 53 geführt und darin ggf. fixierbar.

Beide bügelförmigen Elemente 22 und 34 sind an einem ringförmigen Basiselement 54 befestigt. Das Basiselement 54 ist etwa rund ausgebildet. Die Schwenkachsen 30 und 46 gehen dabei durch das Basiselement 54 hindurch. Dazu ist das erste bügelförmige Element 22 über Achsstifte 56 und 58 im einfachsten Fall mit dem Basiselement 54 verbunden. Entsprechend ist das zweite bügelförmige Element 34 über Achsstifte 60 und 62 mit dem Basiselement 54 verschwenkbar verbunden.

Beim Gebrauch der Vorrichtung 10 wird diese mit dem Basiselement 54 auf die Körperoberfläche 14 aufgelegt. Um eine breitere und kippstabilere Auflage zu erreichen, weist das Basiselement 54 der Körperoberfläche 14 zugewandt eine verbreiterte Auflagefläche 64 auf, die sich vollumfänglich oder teilumfänglich über das Basiselement 54 erstreckt. In Fig. 3 und 4 ist die verbreiterte Auflagefläche 64 nicht dargestellt.

Hinsichtlich der Gewichtsverteilung der Vorrichtung 10 ist es sinnvoll, wenn der Schwerpunkt der Vorrichtung 10, insbesondere wegen der bei eingesetztem Instrument 12 und dessen Schräglage bedingten Kippmomente möglichst nahe der Körperoberfläche 14 liegt, wozu das Basiselement 54 insgesamt mit höherem Gewicht ausgestattet ist als die bügelförmigen Elemente 22 und 34.

Zusätzlich ist das Basiselement 54 auf der Körperoberfläche 14 befestigbar, bspw. klebend durch Verwendung eines doppelseitigen Klebebandes, das einerseits an der Auflagefläche 64 und andererseits auf der Körperoberfläche 14 befestigt ist, oder durch Anlegen eines Vakuums an die Auflagefläche 64, wodurch die Auflagefläche 64 und damit das Basiselement 54 an die Körperoberfläche 14 angesaugt wird.

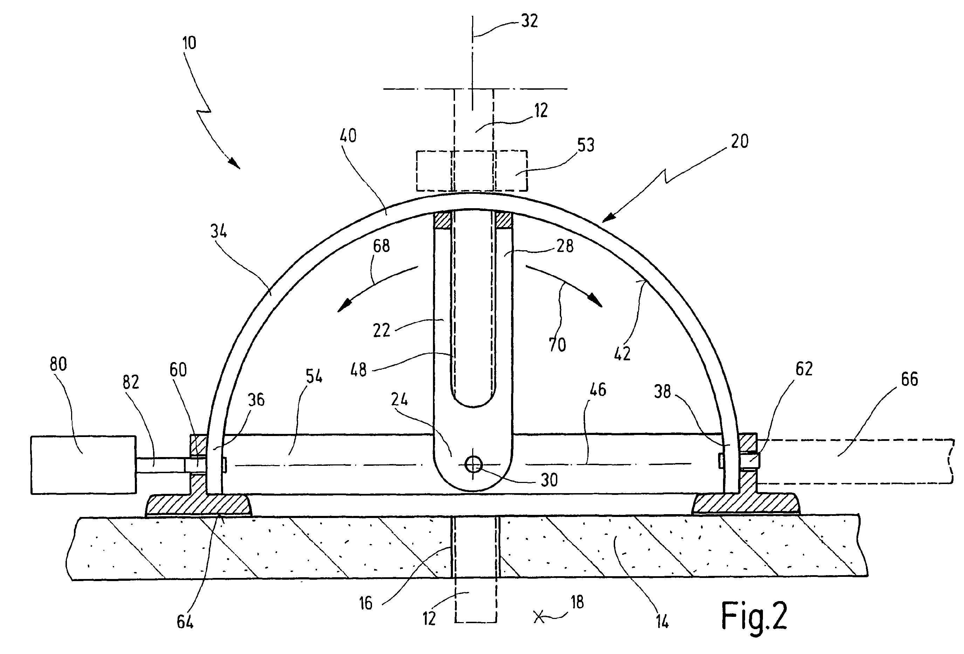

Zur weiteren Stabilisierung der Vorrichtung 10 auf der Körperoberfläche 14 ist das Basiselement 54 mit einem Tragarm eines ortsfesten Tragrahmens 66 verbunden. Als solcher Tragrahmen 66 eignet sich bspw. das in der DE 199 02 036 C1, auf die hier verwiesen wird, in deren Fig. 2 und 3 dargestellte Tragsystem.

Der Halter 20 der Vorrichtung 10 ermöglicht eine Bewegung des Instruments 12 bezüglich der Freiheitsgrade von Schwenkbewegungen um die erste Schwenkachse 30 in Richtung von Pfeilen 68 und 70 in Fig. 2 und um die zweite Schwenkachse 46 in Richtung von Pfeilen 72 und 74. Aufgrund der durch die bügelförmigen Elemente 22 und 34 gebildeten kardanischen Aufhängung kann das Instrument 12 demnach Kippstellungen relativ zur Körperoberfläche 14 bzw. zur Vertikalen 32 in beliebigen Raumwinkeln im Halbraum oberhalb der Körperoberfläche 14 einnehmen. In Fig. 4 ist beispielhaft eine Kippstellung der bügelförmigen Elemente 22 und 34 gezeigt, bei der sowohl das bügelförmige Element 22 als auch das bügelförmige Element 34 aus ihrer in Fig. 1 bis 3 gezeigten vertikalen Stellung verkippt sind.

Während die bügelförmigen Elemente 22 und 34 prinzipiell bezüglich der Schwenkachsen 30 und 46 in einem Winkelbereich von jeweils -90° bis +90° verschwenkbar sind, kann vorgesehen sein, den Schwenkbereich des Instruments 12 bezüglich beider Schwenkachsen auf ± 70° zu begrenzen, um extremale Schrägstellungen des Instruments 12 zu vermeiden. Dies kann durch mechanische Begrenzungen der Verschwenkbarkeit der bügelförmigen Element 22 und 34 erreicht werden, bspw. indem sich die Öffnungen 48 und 50 weniger nahe zu den Endabschnitten 24 und 26 bzw. 36 und 38 hin erstrecken.

Für die Verschwenkung des ersten bügelförmigen Elements 22 ist ein motorischer Antrieb 76 vorgesehen, der mit seiner Abtriebswelle 78 auf die Achse 58 wirkt, um das bügelförmige Element 22 um die erste Schwenkachse 30 zu verschwenken. Entsprechend ist zur Verschwenkung des zweiten bügelförmigen Elements 34 ein motorischer Antrieb 80 vorgesehen, der mit seiner Abtriebswelle 82 auf die Achse 60 wirkt, um das zweite bügelförmige Element 34 um die zweite Schwenkachse 46 zu verschwenken.

Die motorischen Antriebe 76 und 80 weisen jeweils Positionsdetektoren, bspw. in Form von Winkelencodern, auf, um die jeweilige aktuelle Position der bügelförmigen Elemente 22 bzw. 34 bezüglich der Schwenkachsen 30 und 46 zu detektieren. Die motorischen Antriebe 76 und 80 sind als Schrittmotoren ausgeführt. Es ist ferner eine nicht dargestellte Ansteuereinheit zur fortlaufenden Erfassung der Position des Instruments 12 und zur Steuerung der motorischen Antriebe 76 und 80 vorgesehen.

Während die motorischen Antriebe 76 und 80 in Fig. 1 bis 5 außerhalb des durch die bügelförmigen Elemente 22 und 34 aufgespannten Raums angeordnet sind, können die motorischen Antriebe 76 und 80 auch innerhalb dieses Raums angeordnet sein, um eine noch kompaktere Bauweise zu erhalten.

Anstatt die motorischen Antriebe 76 und 80 in axialer Verlängerung der Achsen 58 bzw. 60 anzuordnen, können die motorischen Antriebe 76 und 80 auch beispielsweise vertikal nach oben stehend angeordnet sein, wobei dann die Abtriebswellen 78 bzw. 82 über ein Kegelradgetriebe auf die Achsen 58 und 60 wirken. Diese Anordnung kann dann von Vorteil sein, wenn die Abmessungen der motorischen Antriebe 76 und 80 es bei einer wie in Fig. 1 bis 5 vorgesehenen Anordnung verhindern oder erschweren würden, daß das Basiselement 54 und damit die Schwenkachsen 30 und 46 nicht nahe genug an die Körperoberfläche 14 herangebracht werden können.

Bei der Vorrichtung 10 befinden sich die erste Schwenkachse 30 und die zweite Schwenkachse 46 bereits in unmittelbarer Nähe der Körperoberfläche 14, die wesentlich kleiner ist als der der Zeichnung zu entnehmende Abstand.

Ein Schnittpunkt 84 der ersten Schwenkachse 30 mit der zweiten Schwenkachse 46 definiert den invarianten Punkt der durch die bügelförmigen Elemente 22 und 34 gebildeten kardanischen Aufhängung, d.h. beim Verkippen des Instruments 12 um die erste Schwenkachse 30 und die zweite Schwenkachse 46 kippt das Instrument 12 um den Schnittpunkt 84 als Drehpunkt. Da das Instrument 12 jedoch ebenso eine Führung in der Inzision 16 erfährt, ist es wichtig, den Schnittpunkt 84 möglichst nahe an oder in die Inzision 16 hinein zu verlagern.

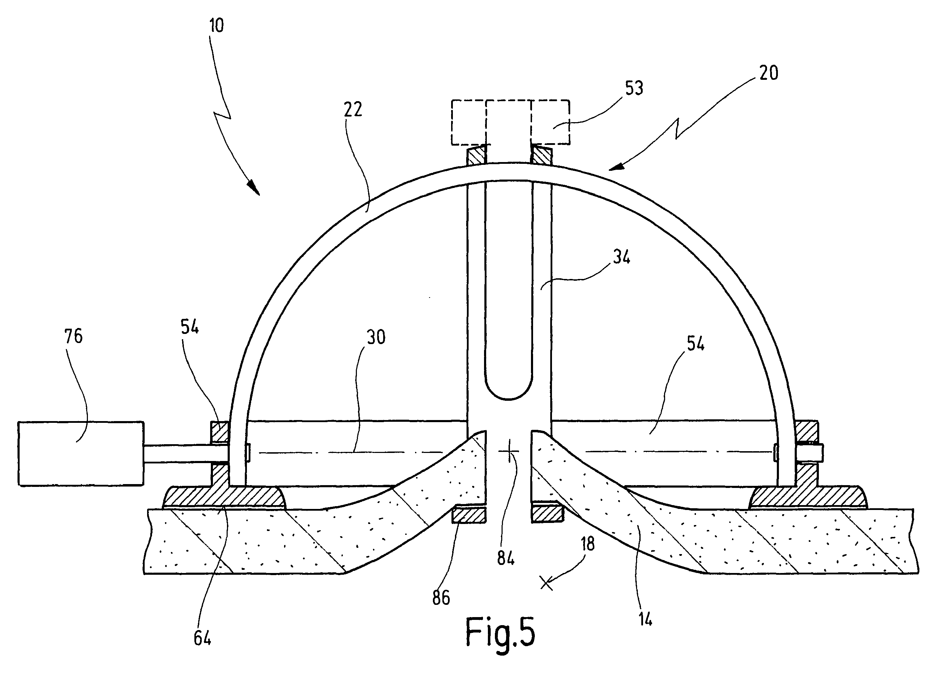

Gemäß Fig. 5 sind bei der Vorrichtung 10 entsprechend Mittel 86 zum Anheben der Körperoberfläche 14 in den von den bügelförmigen Elementen 22 und 34 aufgespannten Raum vorgesehen, derart, daß die Inzision 16 in den Schnittpunkt 84 der beiden Schwenkachsen 30 und 46 verlagert wird.

Die Mittel 86 zum Anheben der Körperoberfläche 14 sind in Fig. 5 in Form eines mechanischen Hebesystems ausgebildet, das bspw. in Form von zwei durch die Inzision 16 hindurchgeführte Greifer gebildet wird, die unter die Körperoberfläche 14 greifen und diese in den durch die bügelförmigen Elemente 22 und 46 aufgespannten Raum anziehen.

Anstelle eines mechanischen Hebesystems kann jedoch auch ein von außen auf die Körperoberfläche 14 wirkender Unterdruck als Mittel zum Anheben der Körperoberfläche 14 eingesetzt werden.

In nicht dargestellter Weise ist der Halter 20 weiterhin so ausgebildet, daß das Instrument 12 in der Führung, die durch den Überlappbereich 52 gebildet wird, um seine Längsachse verdrehbar und/oder in Richtung seiner Längsachse verschiebbar ist. Auf diese Weise werden dem Instrument 12 zusätzlich zu den beiden Freiheitsgraden der Schwenkbewegung um die Schwenkachsen 30 und 46 zwei weitere, vorzugsweise gesteuerte Freiheitsgrade der Bewegung ermöglicht.

Auch für die zuvor genannten beiden zusätzlichen Freiheitsgrade sind vorzugsweise motorische Antriebe am Halter 20 angeordnet, die ebenfalls mit Positionsdetektoren ausgestattet sind, um die aktuelle Position des Instruments 12 bezüglich dieser Freiheitsgrade zu erfassen und über die bereits erwähnte Ansteuereinheit die Position des Instruments 12 zu steuern.

Hinsichtlich der nicht dargestellten motorischen Antriebe für die Verdrehung des Instruments 12 bzw. für die Verschiebung des Instruments 12 ist es vorzugsweise vorgesehen, daß diese motorischen Antriebe indirekt auf das Instrument 12 wirken, bspw. mittels einer Magnetkupplung.

Des weiteren ist für alle zuvor genannten motorischen Antriebe eine Sicherheitsabschaltung vorgesehen, derart, daß durch eine im Instrument 12 induzierte Meßspannung bei Berührung des Instruments 12 mit Gewebe im Körperinneren 18 ein Meßstrom fließt, der eine Abschaltung der motorischen Antriebe 46, 80 und der weiteren motorischen Antriebe für die Verdrehbarkeit und Verschiebbarkeit des Instruments bewirkt.

Des weiteren können die motorischen Antriebe 76 und 80 mit einer Endabschaltung ausgestattet sein, um den Kippbereich des Instruments 12 um die Schwenkachsen 30 und 46 anstatt durch mechanische Vorkehrungen durch die motorische Steuerung zu begrenzen.

## Patentansprüche

1. Vorrichtung zum Halten und Positionieren eines durch eine Körperoberfläche (14) eines Patienten eingeführten endoskopischen Instruments (12), mit einem Halter (20) für das Instrument (12), der so ausgebildet ist, daß das Instrument (12) relativ zur Körperoberfläche (14) in verschied e-nen räumlichen Kippstellungen positionierbar ist, wobei der Halter (20) ein erstes bügelförmiges Element (22) au fweist, dessen beiden Endabschnitte (24, 26) der Körperoberfläche (14) zugewandt angeordnet sind, und dessen mittlerer Abschnitt (28) von der Körperoberfläche (14) a b-gewandt ist, und wobei der Halter (20) ein zweites bügelförmiges Element (34) aufweist, dessen Endabschnitte (36, 38) der Körperoberfläche (14) zugewandt, bezüglich der Endabschnitte (24, 26) des ersten bügelförmigen Elements (22) jedoch um etwa 90° versetzt angeordnet sind, und de s-sen mittlerer Abschnitt (40) von der Körperoberfläche (14) mit im wesentlichen gleichem Abstand wie das erste bügelförmige Element (22) abgewandt ist, und wobei das Instrument (12) an einem Überlappbereich (52) der beiden bügelförmigen Elemente (22, 34) geführt ist, wobei das erste bügelförmige Element (22) um eine erste Schwenkachse (30), die durch beide Endabschnitte (24, 26) des ersten bügelförmigen Elements (22) hindurchgeht, verschwenkbar ist, und das zweite bügelförmige Element (34) um eine zweite Schwenkachse (46), die durch beide Endabschnitte (36, 38) des zweiten bügelförmigen Elements (34) hindurchgeht, verschwenkbar ist, **dadurch gekennzeichnet, daß** Mittel (86) zum Anheben der Körperoberfläche (14) in den von den bügelförmigen Elementen (22, 34) aufgespannten Raum vorgesehen sind.

2. Vorrichtung nach Anspruch 1, **dadurch gekennzeichnet, daß** die bügelförmigen Elemente (22, 34) mit ihren Endabschnitten (24, 26, 36, 38) an einem ringförmigen Basiselement (54) befestigt sind, das auf die Körperoberfläche (14) auflegbar ist, wobei die Schwenkachsen (30, 46) im Basiselement (54) liegen.

3. Vorrichtung nach Anspruch 1 oder 2, **dadurch gekennzeichnet, daß** die Mittel (86) zum Anheben der Körperoberfläche (14) ein mechanisches Hebesystem aufweisen, das beispiel s-weise einen Greifer oder Gewebehaken aufweist.

4. Vorrichtung nach einem der Ansprüche 1 bis 3, **dadurch gekennzeichnet, daß** die Mittel (86) zum Anheben der Körperoberfläche (14) in einem von außen auf die Körperoberfläche (14) applizierten Unterdruck zum Anheben der Körperoberfläche (14) bestehen.

5. Vorrichtung nach einem der Ansprüche 1 bis 4, **dadurch gekennzeichnet, daß** sich die erste Schwenkachse (30) und die zweite Schwenkachse (46) in unmittelbarer Nähe der Körperoberfläche (14) befinden.

6. Vorrichtung nach einem der Ansprüche 1 bis 5, **dadurch gekennzeichnet, daß** die bügelförmigen Elemente (22, 34) als gekrümmte flache Stäbe ausgebildet sind.

7. Vorrichtung nach einem der Ansprüche 2 bis 6, **dadurch gekennzeichnet, daß** das Basiselement (54) schwerer ist als die bügelförmigen Elemente (22, 34).

8. Vorrichtung nach einem der Ansprüche 2 bis 7, **dadurch gekennzeichnet, daß** das Basiselement (54) auf der Körperoberfläche (14) befestigbar ist.

9. Vorrichtung nach Anspruch 8, **dadurch gekennzeichnet, daß** das Basiselement (54) auf der Körperoberfläche (14) klebend befestigbar ist.

10. Vorrichtung nach Anspruch 8 oder 9, **dadurch gekennzeichnet, daß** das Basiselement (54) an der Körperoberfläche (14) durch Anlegen eines Vakuums ansaugbar ist.

11. Vorrichtung nach einem der Ansprüche 2 bis 10, **dadurch gekennzeichnet, daß** das Basiselement (54) mit einem ortsfesten Tragrahmen (66) verbunden ist.

12. Vorrichtung nach einem der Ansprüche 1 bis 11, **dadurch gekennzeichnet, daß** beide bügelförmigen Elemente (22, 34) jeweils eine sich längs der Elemente (22, 34) erstreckende Öffnung (48, 50) aufweisen, und der Überlappbereich (52) der beiden Öffnungen (48, 50) als Führung bzw. Durchführung für das Instrument (11) dient.

13. Vorrichtung nach Anspruch 12, **dadurch gekennzeichnet, daß** die bügelförmigen Elemente (22, 34) etwa halbkreisförmig ausgebildet sind und sich die Öffnungen (48, 50) über nahezu die gesamte Länge der bügelförmigen Elemente (22, 34) erstrecken.

14. Vorrichtung nach einem der Ansprüche 1 bis 13, **dadurch gekennzeichnet, daß** die Verkippung ,der bügelförmigen Elemente (22, 34) auf weniger als ± 90°, vorzugsweise auf ± 70°, bezüglich der Vertikalen (32) begrenzbar ist.

15. Vorrichtung nach einem der Ansprüche 1 bis 14, **dadurch gekennzeichnet, daß** für beide bügelförmigen Elemente (22, 34) ein motorischer Antrieb (76, 80) vorgesehen ist, um die bügelförmigen Elemente (22, 34) um die erste bzw. zweite Schwenkachse (30, 46) zu verschwenken.

16. Vorrichtung nach Anspruch 15, **dadurch gekennzeichnet, daß** die motorischen Antriebe (76, 80) jeweils Positionsdetektoren aufweisen.

17. Vorrichtung nach Anspruch 15 oder 16, **dadurch gekennzeichnet, daß** die motorischen Antriebe innerhalb des von den bügelförmigen Elementen aufgespannten Raums angeordnet sind.

18. Vorrichtung nach einem der Ansprüche 1 bis 17, **dadurch gekennzeichnet, daß** der Halter (20) so ausgebildet ist, daß das Instrument (12) in der Führung der bügelförmigen Elemente (22, 34) um seine Längsachse verdrehbar und/oder in Richtung seiner Längsachse verschiebbar ist.

19. Vorrichtung nach Anspruch 18, **dadurch gekennzeichnet, daß** für die Verdrehbarkeit des Instruments (12) um die Längsachse und/oder für die Verschiebbarkeit des Instruments (12) in Richtung der Längsachse zumindest ein motorischer Antrieb am Halter (20) angeordnet ist.

20. Vorrichtung nach Anspruch 19, **dadurch gekennzeichnet, daß** der zumindest eine motorische Antrieb für die Verdrehung des Instruments (12) bzw. für die Verschiebung des Instruments (12) indirekt auf das Instrument (12) wirkt, vorzugsweise mittels einer Magnetkupplung.

21. Vorrichtung nach einem der Ansprüche 15 bis 17, 19 oder 20, **dadurch gekennzeichnet, daß** die motorischen Antriebe (76, 80) als Schrittmotoren ausgeführt sind.

22. Vorrichtung nach einem der Ansprüche 15 bis 17 oder 19 bis 21, **dadurch gekennzeichnet, daß** für die motorischen Antriebe (76, 80) eine Sicherheitsabschaltung vorgesehen ist, derart, daß durch eine im Instrument (12) induzierte Meßspannung bei Berührung des Instruments (12) mit Gewebe im Körperinneren ein Meßstrom fließt, der eine Abschaltung der motorischen Antriebe (76, 80) bewirkt.

23. Vorrichtung nach einem der Ansprüche 15 bis 17 oder 19 bis 22, weiterhin **gekennzeichnet durch** eine Ansteuereinheit zur fortlaufenden Erfassung der Position des Instruments (12) und Steuerung der motorischen Antriebe (76, 80).

## Claims

1. A device for holding and positioning an endoscopic instrument (12) introduced through a body surface (14) of a patient, comprising a holder (20) for the instrument (12), which holder (20) is designed in such a way that the instrument (12) can be positioned relative to the body surface (14) in different spatial tilt positions, which holder (20) has a first bow-shaped element (22) whose two end portions (24, 26) are arranged directed towards the body surface (14), and whose middle portion (28) is directed away from the body surface (14), and which holder (20) has a second bow-shaped element (34) whose end portions (36, 38) are directed towards the body surface (14), but arranged offset by approximately 90° relative to the end portions (24, 26) of the first bow-shaped element (22), and whose middle portion (40) is directed away from the body surface (14) by substantially the same distance as the first bow-shaped element (22), and the instrument (12) being guided at an overlapping region (52) of the two bow-shaped elements (22, 34), wherein the first bow-shaped element (22) is pivotable about a first pivot axis (30) which extends through both end portions (24, 26) of the first bow-shaped element (22), and the second bow-shaped element (34) is pivotable about a second pivot axis (46) which extends through both end portions (36, 38) of the second bow-shaped element (34), **characterized in that** means (86) are provided for lifting the body surface (14) into the space spanned by the bow-shaped elements (22, 34).

2. The device of claim 1, **characterized in that** the bow-shaped elements (22, 34) are secured with their end portions (24, 26, 36, 38) on an annular base element (54) which can be placed on the body surface (14), the pivot axes (30, 46) lying in the base element (54).

3. The device of claim 1 or 2, **characterized in that** the means (86) for lifting the body surface (14) comprise a mechanical lifting system, which, for example, comprises a gripper or tissue hook.

4. The device of claim 1 or 2, **characterized in that** the means (86) for lifting the body surface (14) consist of an underpressure applied to the body surface from the outside for lifting the body surface (14).

5. The device of anyone of claims 1 through 4, **characterized in that** the first pivot axis (30) and the second pivot axis (46) are situated in immediate proximity to the body surface (14).

6. The device of anyone of claims 1 through 5, **characterized in that** the bow-shaped elements (22, 34) are designed as curved flat bars.

7. The device of anyone of claims 2 through 6, **characterized in that** the base element (54) is heavier than the bow-shaped elements (22, 34).

8. The device of anyone of claims 2 through 7, **characterized in that** the base element (54) can be secured to the body surface (14).

9. The device of claim 8, **characterized in that** the base element (54) can be adhesively secured to the body surface (14).

10. The device of claim 8 or 9, **characterized in that** the base element (54) can be suctioned onto the body surface (14) by applying a vacuum.

11. The device of anyone of claims 2 through 10, **characterized in that** the base element (54) is connected to a fixed support frame (66).

12. The device of anyone of claims 1 through 11, **characterized in that** both bow-shaped elements (22, 34) each have an opening (48, 50) which extends along the elements (22, 34), and the overlap region (52) of the two openings (48, 50) serves as a guide or passage for the instrument (12).

13. The device of claim 12, **characterized in that** the bow-shaped elements (22, 34) are designed approximately in the shape of a semicircle, and the openings (48, 50) extend along almost the entire length of the bow-shaped elements (22, 34).

14. The device of anyone of claims 1 through 13, **characterized in that** the tilting of the bow-shaped elements (22, 34) can be limited to less than ±90°, preferably to ±70°, relative to the vertical (32).

15. The device of anyone of claims 1 through 14, **characterized in that** a motor drive (76, 80) is provided for both bow-shaped elements (22, 34) in order to pivot said bow-shaped elements (22, 34) about the first and second pivot axes (30, 46).

16. The device of claim 15, **characterized in that** the motor drives (76, 80) each have position detectors.

17. The device of claim 15 or 16, **characterized in that** the motor drives are arranged inside the space spanned by the bow-shaped elements (22, 34).

18. The device of anyone of claims 1 through 17, **characterized in that** the holder (20) is designed in such a way that the instrument (12) is rotatable about its longitudinal axis in the guide of the bow-shaped elements (22, 34) and/or is displaceable in the direction of its longitudinal axis.

19. The device of claim 18, **characterized in that** at least one motor drive is arranged on the holder (20) for the rotatability of the instrument (12) about the longitudinal axis and/or for the displaceability of the instrument (12) in the direction of the longitudinal axis.

20. The device of claim 19, **characterized in that** the at least one motor drive for the rotation of the instrument (12) and/or for the displacement of the instrument (12) acts indirectly on the instrument (12), preferably by way of a magnetic coupling.

21. The device of anyone of claims 15 through 17, 19 or 20, **characterized in that** the motor drives (76, 80) are designed as step motors.

22. The device of anyone of claims 15 through 17 or 19 through 21, **characterized in that** a safety switch-off is provided for the motor drives (76, 80) so that, by means of a measurement voltage induced in the instrument (12) upon contact of the instrument (12) with tissue inside the body, a measurement current flows which switches off the motor drives (76, 80).

23. The device of anyone of claims 15 through 17 or 19 through 22, further **characterized by** a control unit for continuously determining the position of the instrument (12) and for controlling the motor drives (76, 80).

## Revendications

1. Dispositif de support et de positionnement d'un instrument endoscopique (12) introduit par une surface du corps (14) d'un patient, avec un support (20) pour l'instrument (12), conçu de façon à ce que l'instrument (12) puisse être positionné dans différentes positions inclinées dans l'espace par rapport à la surface du corps (14), le support (20) comprenant un premier élément en forme d'étrier (22) dont les deux extrémités (24, 26) sont orientées vers la surface du corps (14) et dont la partie centrale (28) est orientée du côté opposé à la surface du corps (14) et le support (20) comprenant un deuxième élément en forme d'étrier (34) dont les extrémités (36, 38) sont orientées du côté de la surface du corps (14) mais décalées, par rapport aux extrémités (24, 26) du premier élément en forme d'étrier (22), d'environ 90°, et dont la partie centrale (40) est orientée du côté opposé à la surface du corps (14) avec le même intervalle que le premier élément en forme d'étrier (22), et l'instrument (12) étant guidé au niveau d'une zone de chevauchement (52) des deux éléments en forme d'étrier (22, 34), le premier élément en forme d'étrier (22) pivotant autour d'un premier axe de pivotement (30), qui traverse les deux extrémités (24, 26) du premier élément en forme d'étrier (22), et le deuxième élément en forme d'étrier (34) pivote autour d'un deuxième axe de pivotement (46) qui traverse les deux extrémités (36, 38) du deuxième élément en forme d'étrier (34), **caractérisé en ce que** des moyens de soulèvement (86) de la surface du corps (14) dans l'espace délimité par les éléments en forme d'étrier (22, 34) sont prévus.

2. Dispositif selon la revendication 1, **caractérisé en ce que** les éléments en forme d'étrier (22, 34) sont fixés, avec leurs extrémités (24, 26, 36, 38), à un élément de base annulaire (54) qui peut être posé sur la surface du corps (14), moyennant quoi les axes de pivotement (30, 46) se trouvent dans l'élément de base (54).

3. Dispositif selon la revendication 1 ou 2, **caractérisé en ce que** les moyens (86) pour soulever la surface du corps (14) comprennent un système de levage mécanique qui comprend par exemple une pince ou un crochet à tissus.

4. Dispositif selon l'une des revendications 1 à 3, **caractérisé en ce que** les moyens (86) pour soulever la surface du corps (14) consistent en une pression négative appliquée de l'extérieur à la surface du corps (14) pour soulever la surface du corps (14).

5. Dispositif selon l'une des revendications 1 à 4, **caractérisé en ce que** le premier axe de pivotement (30) et le deuxième axe de pivotement (46) se trouve à proximité immédiate de la surface du corps (14).

6. Dispositif selon l'une des revendications 1 à 5, **caractérisé en ce que** les éléments en forme d'étrier (22, 34) sont conçus comme des tiges plates incurvées.

7. Dispositif selon l'une des revendications 2 à 6, **caractérisé en ce que** l'élément de base (54) est plus lourd que les éléments en forme d'étrier (22, 34).

8. Dispositif selon l'une des revendications 2 à 7, **caractérisé en ce que** l'élément de base (54) peut être fixé sur la surface du corps (14).

9. Dispositif selon la revendication 8, **caractérisé en ce que** l'élément de base (54) peut être fixé par collage sur la surface du corps (14).

10. Dispositif selon la revendication 8 ou 9, **caractérisé en ce que** l'élément de base (54) peut être fixé à la surface du corps (14) par l'application d'un vide.

11. Dispositif selon l'une des revendications 2 à 10, **caractérisé en ce que** l'élément de base (54) est relié à un châssis de support fixe (66).

12. Dispositif selon l'une des revendications 1 à 11, **caractérisé en ce que** les deux éléments en forme d'étrier (22, 34) comprennent chacun une ouverture (48, 50) qui s'étend le long des éléments (22, 34) et la zone de chevauchement (52) des deux ouvertures (48, 50) sert de guidage ou de passage à l'instrument (12).

13. Dispositif selon la revendication 12, **caractérisé en ce que** les éléments en forme d'étrier (22, 34) ont approximativement une forme d'arc de cercle et les ouvertures (48, 50) s'étendent sur presque toute la longueur des éléments en forme d'étrier (22, 34).

14. Dispositif selon l'une des revendications 1 à 13, **caractérisé en ce que** le pivotement des éléments en forme d'étrier (22, 34) peut être limité à moins de ± 90°, de préférence à ± 70° par rapport à ia verticale (32).

15. Dispositif selon l'une des revendications 1 à 14, **caractérisé en ce que**, pour les deux éléments en forme d'étrier (22, 34), un entraînement motorisé (76, 80) est prévu pour pivoter les éléments en forme d'étrier (22, 34) autour du premier ou du deuxième axe de pivotement (30, 46).

16. Dispositif selon la revendication 15, **caractérisé en ce que** les entraînements motorisés (76, 80) comprennent chacun des détecteurs de position.

17. Dispositif selon la revendication 15 ou 16, **caractérisé en ce que** les entraînements motorisés sont disposés à l'intérieur de l'espace délimité par les éléments en forme d'étrier.

18. Dispositif selon l'une des revendications 1 à 17, **caractérisé en ce que** le support (20) est conçu de façon à ce que l'instrument (12) puisse tourner dans le guidage des éléments en forme d'étrier (22, 34) autour de son axe longitudinal et/ou coulisser en direction de son axe longitudinal.

19. Dispositif selon la revendication 18, **caractérisé en ce que** pour la rotation de l'instrument (12) autour de l'axe longitudinal et/ou pour le coulissement de l'instrument (12) en direction de l'axe longitudinal, au moins un entraînement motorisé se trouve sur le support (20).

20. Dispositif selon la revendication 19, **caractérisé en ce que** l'entraînement motorisé pour la rotation de l'instrument (12) ou pour le coulissement de l'instrument (12) agit de préférence indirectement sur l'instrument (12) par l'intermédiaire d'un couplage magnétique.

21. Dispositif selon l'une des revendications 15 à 17, 19 ou 20, **caractérisé en ce que** les entraînements motorisé (76, 80) sont des moteurs pas à pas.

22. Dispositif selon l'une des revendications 15 à 17, ou 19 à 21, **caractérisé en ce que**, pour les entraînements motorisés (76, 80) un arrêt de sécurité est prévu de façon à ce que, du fait d'une tension de courant induite dans l'instrument (12) lors du contact de l'instrument (12) avec les tissus de l'intérieur du corps, un courant de mesure s'écoule, qui provoque un arrêt des entraînements motorisés (76, 80).

23. Dispositif selon l'une des revendications 15 à 17, ou 19 à 22, **caractérisé en outre par** une unité de commande pour la détermination en continu de la position de l'instrument (12) et pour la commande des entraînements motorisés (76, 80).
